# FASCICULE DE BREVET EUROPEEN

(11) **EP 1 542 944 B1**
(45) Date de publication et mention de la délivrance du brevet: **14.02.2007**
(21) Numéro de dépôt: 03773806.9
(22) Date de dépôt: 23.09.2003
(51) Int. Cl.: C07B 45/06, C07C 319/04, C07C 321/04

(54) **PROCEDE CATLYTIQUE DE FABRICATION D'ALKYLMERCAPTANS APR ADDITION D'HYDROGENE SULFURE SUR UNE OLEFINE**
KATALYTISCHES VERFAHREN ZUR HERSTELLUNG VON ALKYLMERCAPTANEN DURCH ADDITIONSREAKTION VON WASSERSTOFFSULFID AN EINEM OLEFIN
CATALYTIC METHOD OF PRODUCING ALKYL MERCAPTANS BY ADDING HYDROGEN SULPHIDE TO AN OLEFIN

(30) Priorité: 25.09.2002 FR 0211923
(43) Date de publication de la demande: 22.06.2005
(73) Titulaire: Arkema France, 92800 Puteaux (FR)
(72) Inventeur: FREMY, Georges, F-64390 Sauveterre de Bearn (FR); ESSAYEM, Nadine, F-38540 SAINT JUST CHALEYSSIN (FR); LACROIX, Michel, F-69009 Lyon (FR); ZAUSA, Elodie, F-33760 Targon (FR)
(74) Mandataire: Treuil, Claude
(86) Numéro de dépôt international: PCT/FR2003/002789
(87) Numéro de publication internationale: WO 2004/029005

(56) Documents cités:
- EP-A- 0 354 460
- US-A- 3 036 133

## Description

La présente invention concerne le domaine des mercaptans (encore appelés thiols) et a plus particulièrement pour objet un procédé catalytique pour la fabrication de mercaptans à partir d'une oléfine et d'hydrogène sulfuré, en présence d'hydrogène et d'un catalyseur spécifique.

L'intérêt industriel des mercaptans ou thiols fait que de nombreux travaux ont été effectués en vue de la mise au point de la fabrication de ces composés.

On connaît notamment un procédé largement employé qui met en oeuvre la réaction de l'hydrogène sulfuré avec un alcool ou une oléfine. Dans une telle réaction, on obtient en particulier comme sous-produit un ou plusieurs thioéthers qui résultent de réactions secondaires, et principalement de la réaction du mercaptan (formé dans la réaction principale) sur le réactif de départ c'est-à-dire soit l'alcool, soit l'oléfine, selon le procédé utilisé.

Les thioéthers obtenus en tant que sous-produits lors de la fabrication de mercaptans n'ont généralement pas d'intérêt commercial.

Il est donc essentiel d'améliorer la sélectivité du catalyseur mis en oeuvre dans la réaction, de manière à augmenter le rendement en thiol, plus particulièrement lorsque ce dernier est obtenu par addition d'hydrogène sulfuré sur une oléfine, réaction souvent appelée sulfhydratation.

Des méthodes de sulfhydratation ont été proposées, visant à faire réagir sous pression de l'hydrogène sulfuré (H₂S) sur une oléfine en présence de différents catalyseurs.

Bien des catalyseurs ont été proposés dans l'art antérieur, notamment de l'acide phosphorique supporté (par l'US 2950324), de la silice avec de faibles quantités d'alumine (par l'US 2951875), une zéolite synthétique (par les US 4102931 et US 5453544) ou une résine échangeuse d'ions (cf US 4102931). Les résines échangeuses d'ions permettent d'obtenir des valeurs intéressantes pour la conversion de l'oléfine, et la sélectivité en mercaptan. Toutefois ces résines se dégradent à partir de 100°C, et sont totalement décomposées à 140°C. Il en résulte qu'elles ne permettent pas de catalyser les réactions de sulfhydratation qui, en raison de l'oléfine utilisée, nécessitent une température élevée.

Le brevet US 6162952 décrit un catalyseur supporté sur un oxyde (TiO₂ ou ZrO₂) associé à un site acide H₂SO₄, (NH₄)₂SO₄ ou WO₃. Cependant en raison de la stabilité également limitée de ces solides en température (à environ 200°C), ce catalyseur présente le même inconvénient que précédemment.

Le brevet US 3036133 décrit la préparation de l'éthylmercaptan et du diéthylsulfure par addition de l'H₂S sur l'éthylène, en présence d'un catalyseur comprenant de la silice ou de l'alumine activée par un hétéropolyacide, ou un de ses sels alcalins ou alcalino-terreux. Cependant ce catalyseur donne lieu, lorsqu'il est employé pour une oléfine autre que l'éthylène, par exemple le propène ou le butène, à une conversion en oléfine faible. De plus la sélectivité, et donc le rendement en mercaptan est généralement insuffisant.

Un catalyseur solide comprenant un acide 12-phosphotungstique supporté sur silice est également décrit par le brevet US 5,420,092. Ce document enseigne, plus généralement, la mise en oeuvre d' un hétéropolyacide combiné à un métal du groupe VIII, mais dans le domaine éloigné de l'isomérisation des paraffines.

Il a maintenant été trouvé un nouveau procédé catalytique pour la préparation de mercaptans à partir d'oléfines et d'hydrogène sulfuré qui met en oeuvre de l'hydrogène dans le flux réactionnel et un catalyseur spécifique. Il présente l'avantage de pouvoir être utilisé à une température plus haute, d'obtenir un rendement amélioré pour le mercaptan désiré, et de maintenir une activité élevée pour le catalyseur au cours du temps.

L'invention a donc pour objet un procédé de fabrication d'un mercaptan à partir d'une oléfine et d'hydrogène sulfuré, caractérisé en ce qu'il est effectué en présence d'hydrogène et d'une composition catalytique comprenant un acide fort et au moins un métal appartenant au groupe VIII de la classification périodique.

La combinaison de l'hydrogène avec cette composition catalytique permet de stabiliser l'activité du catalyseur dans le temps à un haut niveau, et ceci même pour une température d'emploi relativement élevée. Ce résultat est d'autant plus surprenant qu'il est obtenu dans un milieu sulfurant, connu pour empoisonner les sites actifs des catalyseurs.

L'acide fort utilisable dans la composition catalytique est pris dans le groupe comprenant :
- (a) un ou plusieurs hétéropolyacide(s) choisi(s) parmi :
   - (i) un composé de formules : H₃PW₁₂O₄₀,nH₂O, H₄SiW₁₂O₄₀,nH₂O ou H₆P₂W₁₈O₆₂,nH₂O dans lesquelles n est un nombre entier représentant le nombre de molécules d'eau de cristallisation, et (s'agissant d'un produit commercial) est généralement compris entre 0 et 30, de préférence entre 6 et 20 ;
   - (ii) un sel de potassium, rubidium, césium ou d'ammonium d'au moins un composé (i) ou un mélange de tels sels ;
- (b) une zircone sulfatée,
- (c) une zircone tungstée,
- (d) une zéolithe, et
- (e) une résine cationique.

L' hétéropolyacide (i) est généralement obtenu par la condensation de 2 ou plus oxoacides différents, tels que l'acide phosphorique, l'acide silicique ou l'acide tungstique. Il est soluble dans l'eau ou dans un solvant organique polaire. Le composé de formule : H₃PW₁₂O₄₀,nH₂O est connu sous la dénomination d'acide 12-phosphotungstique ou 12-tungstophosphorique, et est disponible dans le commerce. Le composé de formule : H₄SiW₁₂O₄₀,nH₂O est connu sous le nom d'acide 12-tungstosilicique ou 12-silicotungstique, et est également disponible dans le commerce. Le composé de formule : H₆P₂W₁₈O₆₂,nH₂O peut être préparé selon le mode opératoire décrit dans la référence suivante : A. P. Ginsberg, Inorganic Synthesis, Vol 27, Published by J. Wiley & sons (1990) pages 105-107.

L'hétéropolyacide (ii) est un sel obtenu par substitution partielle d'un ou plusieurs protons de l'hétéropolyacide (i) par le cation correspondant. Il est clair pour l'homme du métier qu'une telle substitution ne peut être totale, sans quoi l'acidité serait perdue. Un tel sel est préparé à partir d'uné solution de l'hétéropolyacide (i) à laquelle est ajoutée la quantité souhaitée du précurseur de l'alcalin ou de l'ammonium. Le précurseur préféré est le chlorure ou le carbonate correspondant. Le sel précipité est séparé, puis séché dans des conditions douces, de préférence par centrifugation suivie d'une lyophilisation. On peut citer comme référence: N. Essayem, G. Coudurier, M. Fournier, J.C. Vedrine, *Catal*. *Lett.,* 34 (1995) pages 224-225.

La zircone sulfatée (b) est préparée par imprégnation d'acide sulfurique sur un support d'oxyde de zirconium conformément au procédé décrit dans la référence : F. R. Chen, G. Coudurier, J-F Joly and J.C. Vedrine, *J*. *Catal.,* 143 (1993) page 617.

La zircone tungstée (c) est préparée par imprégnation d'oxyde de tungstène sur un support d'oxyde de zirconium, conformément au procédé décrit dans le brevet Soled et al. US 5113034.

Selon une première variante de réalisation du procédé selon l'invention, le catalyseur mis en oeuvre dans celui-ci comprend comme acide fort un hétéropolyacide (ii), ou l'un des composés (b), (c), (d) ou (e). Cette variante est préférée car, en raison des propriétés de surface spécifique d'un tel acide fort, ce dernier convient généralement comme support. Il n'est donc pas dans ce cas nécessaire de déposer l'acide fort sur un support.

La composition catalytique comprend dans ce cas :
- de 90 à 99,9 %, de préférence de 98,5 à 99,5 %, en poids d'acide fort, et
- de 0,01 à 10 %, de préférence de 0,05 à 1,5 % en poids de métal du groupe VIII.

Selon une deuxième variante de réalisation, le catalyseur mis en oeuvre comprend comme acide fort un hétéropolyacide (i). Cette variante est préférée en raison d'une activité particulièrement avantageuse du catalyseur dans la réaction de sulfhydrolyse.

La composition catalytique comprend dans ce cas :
- de 10 à 60 %, et de préférence de 25 à 50 % en poids d'acide fort,
- de 0,01 à 10 %, de préférence de 0,1 à 2 % en poids de métal du groupe VIII, et
- de 30 à 80 % de préférence de 48 à 75 % en poids d'un support choisi parmi la silice SiO₂, l'alumine Al₂O₃, l'oxyde de titane TiO₂, la zircone ZrO₂ ou du charbon actif.

Selon un mode de réalisation particulièrement préféré, l'acide fort mis en oeuvre dans le catalyseur est l'acide 12-phosphotungstique, imprégné de préférence sur de la silice.

Le ou les métaux appartenant au groupe VIII de la classification périodique généralement compris dans la composition catalytique mise en oeuvre sont choisis parmi notamment le fer, le cobalt, le nickel, le ruthénium, le rhodium, le palladium, l'osmium, l'iridium, le platine.

On préfère employer un métal du groupe VIII choisi parmi le palladium, le ruthénium et le platine, et tout particulièrement le platine.

Une composition catalytique particulièrement préférée est celle comprenant : 40 % en poids d'acide 12-phosphotungstique, 1 % de platine et 59 % de silice.

La composition catalytique mise en oeuvre dans le procédé selon l'invention peut être préparée de façon générale de la manière suivante.

Lorsque l'acide fort utilisé est l'un des composés (i), on procède à :
- (1) un traitement thermique du support sous vide à une température comprise entre 90 et 150°C, de préférence autour de 100°C, puis
- (2) une imprégnation du support ainsi traité avec une solution aqueuse ou organique de pH acide contenant le composé (i) et un précurseur acide du métal du groupe VIII, puis
- (3) un séchage du solide ainsi obtenu, puis
- (4) un traitement par H₂ à une température comprise entre 80 et 300°C, de préférence entre 180 et 250 °C.

Le traitement thermique de l'étape (1) a pour but de désorber l'eau éventuellement adsorbée dans les pores du support.

Dans l'étape (2), on entend par précurseur acide un composé donnant en solution aqueuse un complexe cationique ou anionique dudit métal. Des exemples de tels composés sont, dans le cas du platine : l'hydroxyde de platine tétramine, le chlorure de platine tétramine, le dinitrodiamine-platine (II), ou encore, dans le cas du palladium, le chlorure de palladium, Pd(NH₃)₄Cl₂, (NH₄)₂(PdCl₄). Des exemples de tels composés sont encore, dans le cas du platine : l'acide hexachloroplatinique (encore appelé hexachloroplatinate (IV) d'hydrogène), le tetrachloroplatinate (II) d'ammonium, l'hexachloroplatinate (IV) d'ammonium. La liste des précurseurs acides est donnée précédemment à titre purement illustratif, sans limiter les composés utilisables comme précurseur acide par l'homme du métier.

Dans l'étape (3), le séchage peut être par exemple réalisé en chauffant le support imprégné, éventuellement sous vide, à une température généralement comprise entre la température ambiante et 120 °C, durant un temps allant de 30 minutes à 5 heures.

Le traitement par H₂ de l'étape (4) est avantageusement réalisé sur le catalyseur lorsque ce dernier est placé dans le réacteur de sulfhydrolyse, et a pour but la réduction du précurseur acide en métal du groupe VIII.

Lorsque le catalyseur mis en oeuvre comprend comme acide fort un hétéropolyacide (ii), ou l'un des composés (b), (c), (d) ou (e), il peut être préparé selon le même procédé, à l'exception du fait que le traitement thermique n'est pas obligatoire, et doit même être supprimé ou modifié en fonction des caractéristiques du support.

La composition catalytique décrite précédemment est mise en oeuvre dans le procédé de fabrication de mercaptan selon l'invention qui comprend la réaction d'hydrogène sulfuré (H₂S) sur une oléfine en présence d'hydrogène.

Ce procédé est effectué en phase gaz, dans la mesure où les conditions de température et de pression utilisées sont telles que les réactifs et les produits sont à l'état gazeux.

L'hydrogène est introduit dans le procédé à raison d'une quantité correspondant à un rapport molaire H₂S / H₂ compris entre 0,05 et 200, de préférence entre 0,1 et 100.

L'oléfine utilisée comme réactif de départ a pour formule générale :

R₁R₂C=CR₃R₄ (I)

dans laquelle R₁, R₂, R₃, R₄, identiques ou différents, représentent un atome d'hydrogène ou un radical alkyle de 1 à 20 atomes de carbone, de préférence de 1 à 12 atomes de carbone, linéaire ou ramifié.

L'oléfine préférentiellement utilisée est l'éthylène. La réaction de sulfhydratation conduit dans ce cas à l'éthylmercaptan (ou éthanethiol).

L'hydrogène sulfuré est introduit dans le procédé en quantité suffisante pour obtenir la conversion de l'oléfine. En général cette quantité correspond à un rapport molaire H₂S / oléfine compris entre 1 et 100, de préférence entre 2 et 30, encore plus préférentiellement entre 4 et 12.

Les réactifs décrits ci-dessus sont mis en contact, en présence d'une charge de la composition catalytique définie précédemment, dans une zone réactionnelle adaptée, dans des condtions réactionnelles propres à produire le thiol désiré.

On préfère mettre en oeuvre le procédé dans un réacteur alimenté en continu par les réactifs, mais un réacteur de type batch peut également être utilisé.

La température de réaction varie selon l'oléfine utilisée et le degré de conversion désiré, mais se situe généralement dans un domaine compris entre 30 et 350°C, de préférence entre 50 et 250°C.

La pression à laquelle la réaction est réalisée varie également dans de larges limites. Habituellement, elle se situe entre la pression atmosphérique et 50 bars, de préférence entre la pression atmosphérique et 15 bars.

Le temps de contact est généralement compris entre 1 et 50 s, de préférence entre 2 et 30 s.

Les exemples ci-après sont donnés à titre purement illustratifs de l'invention, et ne doivent nullement être interprétés comme une limitation de celle-ci. Dans ces exemples l'abréviation HPW correspond à l'acide 12-phosphotungstique de formule H₃PW₁₂O₄₀,nH₂O.

### Exemple 1 (préparatoire): Préparation du catalyseur Pt et HPW supportés sur SiO₂

Pour 200 g de SiO₂, on prépare une solution aqueuse comprenant 7,5 g de l'acide hexachloroplatinique, de formule H₂PtCl₆ et 140 g d' HPW (poids exprimé en équivalent acide anhydre, soit avec n égal à 0).

On utilise comme support du catalyseur une silice amorphe ayant une surface spécifique (ou BET) de 315 m².g⁻¹, un diamètre de pores de l'ordre de 12 à 14 nm et un volume poreux de 1,6 cm³.g⁻¹. Ce support est préalablement traité sous vide à une température de 100°C.

L'imprégnation de la solution précédemment obtenue sur le support ainsi traité est réalisée sous vide par aspiration. Une fois la solution imprégnée, le mélange est agité pendant 1 heure à pression atmosphérique.

Le produit obtenu est séché sous vide, à température ambiante, puis est soumis à un traitement par de l'hydrogène à une température de 200°C visant à réduire le platine.

Le catalyseur obtenu est constitué de 59% en poids de SiO₂, 1% en poids de platine et 40% en poids d'HPW.

### Exemple 2 : Préparation de l'éthyl mercaptan (CH₃CH₂-SH) à partir de l'éthylène :

On utilise un microréacteur de 15 mm de diamètre présentant une capacité utile de 5 ml chargé de 1,2 ml (0,1 g) de la composition catalytique préparée selon l'exemple 1.

A travers cette charge, on fait passer par heure, 70 l d'éthylène (soit 3 mole), 270 l d'H₂S (soit 12 mole) et 1700 1 d'H₂ (soit 53 mole).

La pression dans le réacteur est maintenue à pression atmosphérique et la température est fixée à 200°C.

Après avoir atteint l'état stationnaire, on mesure une conversion en oléfine de 3,4% et un rendement en éthyl mercaptan de 3,3%.

### Exemple 3 : Préparation de l'éthyl mercaptan (CH₃CH₂-SH) à partir de l'éthylène - Evolution de la conversion de l'éthyl mercaptan en fonction du temps :

On répète l'exemple 2 en poursuivant la réaction de sulfhydratation pendant 48 heures avec la même charge de composition catalytique, et en procédant périodiquement (en fonction du temps exprimé en heures) à la mesure de la conversion de l'éthylène. Les résultats sont rassemblés dans le tableau 1 ci-dessous.

**Tableau 1**

| **Temps (heures)** | **Conversion de l'éthylène (en %)** |
|---|---|
| 1 | 4,5 |
| 2 | 4,3 |
| 20 | 3,4 |
| 48 | 3,4 |

Le tableau 1 montre que le système catalytique préparé à l'exemple 1 et utilisé en présence d'hydrogène selon le procédé de l'invention a une bonne stabilité au cours du temps.

### Exemple 4 (comparatif) : Préparation de l'éthyl mercaptan (Et-SH) à partir de l'éthylène avec un catalyseur de type Cr₂O₃ supporté sur Al₂O₃

On répète l'exemple 2 en supprimant l'introduction de H₂ et en remplaçant la composition catalytique de l'exemple 1 par un catalyseur de type Cr₂O₃ supporté sur Al₂O₃ (teneur en Cr de 19% en poids). Un tel catalyseur est souvent utilisé dans des conditions industrielles de fabrication, et est utilisé ici à titre de référence.
On mesure, également à l'état stationnaire, une conversion initiale de l'éthylène de 1,3 % et un rendement en éthyl mercaptan de 1,2 %.

## Revendications

1. Procédé de fabrication d'un mercaptan à partir d'une oléfine et d'hydrogène sulfuré, en présence d'une composition catalytique comprenant un acide fort et au moins un métal appartenant au groupe VIII de la classification périodique, **caractérisé en ce qu'**il est effectué en présence d'hydrogène.

2. Procédé selon la revendication 1, **caractérisé en ce que** l'acide fort est pris dans le groupe comprenant :
- (a) un ou plusieurs hétéropolyacide(s) choisi(s) parmi :
- (i) un composé de formules : H₃PW₁₂O₄₀,nH₂O, H₄SiW₁₂O₄₀,nH₂O ou H₆P₂W₁₈O₆₂,nH₂O dans lesquelles n est un nombre entier représentant le nombre de molécules d'eau de cristallisation, et est compris entre 0 et 30, de préférence entre 6 et 20 ;
- (ii) un sel de potassium, rubidium, césium ou d'ammonium d'au moins un composé (i) ou un mélange de tels sels ;
- (b) une zircone sulfatée,
- (c) une zircone tungstée,
- (d) une zéolithe, et
- (e) une résine cationique.

3. Procédé selon la revendication 2, **caractérisé en ce que** l'acide fort est un hétéropolyacide (ii), ou l'un des composés (b), (c), (d) ou (e).

4. Procédé selon la revendication 3, **caractérisé en ce que** la composition catalytique comprend :
- de 90 à 99,9 %, de préférence de 98,5 à 99,5 %, en poids d'acide fort, et
- de 0,01 à 10 %, de préférence de 0,05 à 1,5 % en poids de métal du groupe VIII.

5. Procédé selon la revendication 2, **caractérisé en ce que** l'acide fort est un hétéropolyacide (i).

6. Procédé selon la revendication 5, **caractérisé en ce que** la composition catalytique comprend :
- de 10 à 60 %, et de préférence de 25 à 50 % en poids d'acide fort,
- de 0,01 à 10 %, de préférence de 0,1 à 2 % en poids de métal du groupe VIII, et
- de 30 à 80 % de préférence de 48 à 75 % en poids d'un support choisi parmi la silice SiO₂, l'alumine Al₂O₃, l'oxyde de titane TiO₂, la zircone ZrO₂ ou du charbon actif.

7. Procédé selon l'une des revendications 5 ou 6, **caractérisé en ce que** l'acide fort est l'acide 12-phosphotungstique, imprégné de préférence sur de la silice.

8. Procédé selon l'une des revendications 1 à 7, **caractérisé en ce que** le métal est choisi parmi le fer, le cobalt, le nickel, le ruthénium, le rhodium, le palladium, l'osmium, l'iridium, le platine

9. Procédé selon l'une des revendications 1 à 8, **caractérisé en ce que** le métal est choisi parmi le palladium, le ruthénium et le platine.

10. Procédé selon l'une des revendications 1 à 9, **caractérisé en ce que** le métal est le platine.

11. Procédé selon l'une des revendications 1 ou 5 à 10, **caractérisé en ce que** la composition catalytique comprend : 40 % en poids d'acide 12-phosphotungstique, 1 % de platine et 59 % de silice.

12. Procédé selon l'une des revendications 1 à 11, **caractérisé en ce que** l'hydrogène est introduit à raison d'une quantité correspondant à un rapport molaire H₂S / H₂ compris entre 0,05 et 200, de préférence entre 0,1 et 100.

13. Procédé selon l'une des revendications 1 à 12, **caractérisé en ce que** l'oléfine utilisée a pour formule générale :
R₁R₂C=CR₃R₄ (I)
dans laquelle R₁, R₂, R₃, R₄, identiques ou différents, représentent un atome d'hydrogène ou un radical alkyle de 1 à 20 atomes de carbone, de préférence de 1 à 12 atomes de carbone, linéaire ou ramifié.

14. Procédé selon l'une des revendications 1 à 13, **caractérisé en ce que** l'oléfine utilisée est l'éthylène.

15. Procédé selon l'une des revendications 1 à 14, **caractérisé en ce que** l'hydrogène sulfuré est introduit dans une quantité correspondant à un rapport molaire H₂S / oléfine compris entre 1 et 100, de préférence entre 2 et 30, encore plus préférentiellement entre 4 et 12.

## Claims

1. Process for manufacturing a mercaptan from an olefin and hydrogen sulphide in the presence of a catalytic composition comprising a strong acid and at least one metal belonging to Group VIII of the Periodic Table, **characterized in that** it is carried out in the presence of hydrogen.

2. Process according to Claim 1, **characterized in that** the strong acid is taken from the group comprising:
- (a) one or more heteropolyacids chosen from:
- (i) a compound of formula: H₃PW₁₂O₄₀·nH₂O, H₄SiW₁₂O₄₀·nH₂O or H₆P₂W₁₈O₆₂·nH₂O in which n is an integer representing the number of molecules of water of crystallization, and is between 0 and 30, preferably between 6 and 20;
- (ii) a potassium, rubidium, caesium or ammonium salt of at least one compound (i) or a mixture of such salts;
- (b) a sulphated zirconia;
- (c) a tungstated zirconia;
- (d) a zeolite, and
- (e) a cationic resin.

3. Process according to Claim 2, **characterized in that** the strong acid is a heteropolyacid (ii) or one or compounds (b), (c), (d) or (e).

4. Process according to Claim 3, **characterized in that** the catalytic composition comprises:
- 90 to 99.9%, preferably 98.5 to 99.5%, by weight of strong acid; and
- 0.01 to 10%, preferably 0.05 to 1.5%, by weight of metal of Group VIII.

5. Process according to Claim 2, **characterized in that** the strong acid is a heteropolyacid (i).

6. Process according to Claim 5, **characterized in that** the catalytic composition comprises:
- 10 to 60%, preferably 25 to 50%, by weight of strong acid;
- 0.01 to 10%, preferably 0.1 to 2%, by weight of metal of Group VIII; and
- 30 to 80%, preferably 48 to 75%, by weight of a support chosen from silica SiO₂, alumina Al₂O₃, titanium oxide TiO₂, zirconia ZrO₂ and active carbon.

7. Process according to either of Claims 5 and 6, **characterized in that** the strong acid is 12-phosphotungstic acid, preferably impregnated on silica.

8. Process according to one of Claims 1 to 7, **characterized in that** the metal is chosen from iron, cobalt, nickel, ruthenium, rhodium, palladium, osmium, iridium and platinum.

9. Process according to one of Claims 1 to 8, **characterized in that** the metal is chosen from palladium, ruthenium and platinum.

10. Process according to one of Claims 1 to 9, **characterized in that** the metal is platinum.

11. Process according to one of Claims 1 or 5 to 10, **characterized in that** the catalytic composition comprises, by weight, 40% 12-phosphotungstic acid, 1% platinum and 59% silica.

12. Process according to one of Claims 1 to 11, **characterized in that** the hydrogen is introduced in an amount corresponding to an H₂S/H₂ molar ratio of between 0.05 and 200, preferably between 0.1 and 100.

13. Process according to one of Claims 1 to 12, **characterized in that** the olefin used has the general formula:
R₁R₂C=CR₃R₄ (I)
in which R₁, R₂, R₃, R₄, which are identical or different, represent a hydrogen atom or a linear or branched, alkyl radical containing 1 to 20 carbon atoms, preferably 1 to 12 carbon atoms.

14. Process according to one of Claims 1 to 13, **characterized in that** the olefin used is ethylene.

15. Process according to one of Claims 1 to 14, **characterized in that** the hydrogen sulphide is introduced in an amount corresponding to an H₂S/olefin molar ratio of between 1 and 100, preferably between 2 and 30, and even more preferably between 4 and 12.

## Patentansprüche

1. Verfahren zur Herstellung eines Mercaptans aus einem Olefin und Schwefelwasserstoff in Gegenwart einer katalytischen Zusammensetzung, die eine starke Säure und mindestens ein Metall aus der Gruppe VIII des Periodensystems umfasst, **dadurch gekennzeichnet, dass** es in Gegenwart von Wasserstoff durchgeführt wird.

2. Verfahren nach Anspruch 1, **dadurch gekennzeichnet, dass** die starke Säure aus der Gruppe stammt, die Folgendes umfasst:
- (a) eine oder mehrere Heteropolysäure(n), ausgewählt aus:
- (i) einer Verbindung der folgenden Formeln: H₃PW₁₂O₄₀ · nH₂O, H₄SiW₁₂O₄₀·nH₂O oder H₆P₂W₁₈O₆₂ · nH₂O, worin n eine ganze Zahl ist, welche die Anzahl der Kristallwassermoleküle wiedergibt, und zwischen 0 und 30, vorzugsweise zwischen 6 und 20, liegt;
- (ii) ein Kalium-, Rubidium-, Cäsium- oder Ammoniumsalz mindestens einer Verbindung (i) oder ein Gemisch dieser Salze;
- (b) ein Zirkonsulfat,
- (c) ein Zirkonwolframat,
- (d) ein Zeolith und
- (e) ein kationisches Harz.

3. Verfahren nach Anspruch 2, **dadurch gekennzeichnet, dass** die starke Säure eine Heteropolysäure (ii) oder eine der Verbindungen (b), (c), (d) oder (e) ist.

4. Verfahren nach Anspruch 3, **dadurch gekennzeichnet, dass** die katalytische Zusammensetzung Folgendes umfasst:
- 90 bis 99,9 Gew.-%, vorzugsweise 98,5 bis 99,5 Gew.-%, der starken Säure
und
- 0,01 bis 10 Gew.-%, vorzugsweise 0,05 bis 1,5 Gew.-% des Metalls der Gruppe VIII.

5. Verfahren nach Anspruch 2, **dadurch gekennzeichnet, dass** die starke Säure eine Heteropolysäure (i) ist.

6. Verfahren nach Anspruch 5, **dadurch gekennzeichnet, dass** die katalytische Zusammensetzung Folgendes umfasst:
- 10 bis 60 Gew.-%, vorzugsweise 25 bis 50 Gew.-%, der starken Säure,
- 0,01 bis 10 Gew.-%, vorzugsweise 0,1 bis 2 Gew.-% des Metalls der Gruppe VIII und
- 30 bis 80 Gew.-%, vorzugsweise 48 bis 75 Gew.-% eines Trägers, ausgewählt aus Siliziumdioxid SiO₂, Aluminiumdioxid Al₂O₃, Titanoxid TiO₂, Zirkonoxid ZrO₂ oder Aktivkohle.

7. Verfahren nach einem der Ansprüche 5 oder 6, **dadurch gekennzeichnet, dass** es sich bei der starken Säure um 12-Phosphowolframsäure handelt, die vorzugsweise auf Siliziumdioxid imprägniert ist.

8. Verfahren nach einem der Ansprüche 1 bis 7, **dadurch gekennzeichnet, dass** das Metall aus Eisen, Kobalt, Nickel, Ruthenium, Rhodium, Palladium, Osmium, Iridium, Platin ausgewählt ist.

9. Verfahren nach einem der Ansprüche 1 bis 8, **dadurch gekennzeichnet, dass** das Metall aus Palladium, Ruthenium und Platin ausgewählt ist.

10. Verfahren nach einem der Ansprüche 1 bis 9, **dadurch gekennzeichnet, dass** das Metall Platin ist.

11. Verfahren nach einem der Ansprüche 1 oder 5 bis 10, **dadurch gekennzeichnet, dass** die katalytische Zusammensetzung Folgendes umfasst: 40 Gew. -% 12-Phosphowolframsäure, 1% Platin und 59% Siliziumdioxid.

12. Verfahren nach einem der Ansprüche 1 bis 11, **dadurch gekennzeichnet, dass** Wasserstoff in einer Menge eingebracht wird, die einem Molverhältnis von H₂S/H₂ zwischen 0,05 und 200, vorzugsweise zwischen 0,1 und 100, entspricht.

13. Verfahren nach einem der Ansprüche 1 bis 12, **dadurch gekennzeichnet, dass** das verwendete Olefin die folgende allgemeine Formel hat:
R₁R₂C=CR₃R₄ (I)
worin R₁, R₂, R₃, R₄, die gleich oder verschieden sind, ein Wasserstoffatom oder einen geraden oder verzweigten Alkylrest mit 1 bis 20 Kohlenstoffatomen, vorzugsweise 1 bis 12 Kohlenstoffatomen, darstellen.

14. Verfahren nach einem der Ansprüche 1 bis 13, **dadurch gekennzeichnet, dass** das verwendete Olefin Ethylen ist.

15. Verfahren nach einem der Ansprüche 1 bis 14, **dadurch gekennzeichnet, dass** Schwefelwasserstoff in einer Menge eingebracht wird, die einem Molverhältnis von H₂S/Olefin zwischen 1 und 100, vorzugsweise zwischen 2 und 30, noch stärker bevorzugt zwischen 4 und 12, entspricht.
